# EUROPEAN PATENT APPLICATION

(11) **EP 2 404 634 A1**
(43) Date of publication of application: **11.01.2012**
(21) Application number: 11005444.2
(22) Date of filing: 05.07.2011
(51) Int. Cl.: A61M 15/00, A61M 15/02, A62B 7/02, B65D 83/14, A61M 16/10

(54) **Inhaler of oxygen anions**

(30) Priority: 05.07.2010 SK 56902010 U
(71) Applicant: Humenanský, Frantisek, 08001 Presov (SK); Humenanský, Marcel, 08001 Presov (SK)
(72) Inventor: Humenanský, Frantisek, 08001 Presov (SK); Humenanský, Marcel, 08001 Presov (SK)
(74) Representative: Regina, Ivan

(57) **Abstract**

Inhaler of oxygen anions filled with the medically conditioned oxygen and enriched by negative onions consists of a container (1) with a dosing valve (2), a dose controller (3), an inhaling cap (4) and a shield cover (5).

It allows to apply to the human body oxygen with increased amount of negative ions without harmful amounts of ozone without the need for a power source, whether from the grid power or from battery.

## Description

### Branch of technique

The invention refers to a portable, small - capacity inhaler negative ions of oxygen (anions) included in molecules of medical oxygen (O₂). The inhalation based on positive effects of negative ions of oxygen and oxygen itself in general on human body enables compensation of a lack of physical and mental energy of human body in different loading situations.

### The present state of technique

The importance of balance between negative and positive ions in air for the human body has been already proved. In the fresh air in nature negative and positive ions are in an approximate balance - ratio of 750 positive to 650 negative ions in 1 cm³ (so called unipolar coefficient q=750/650=1,15). An excess of positive ions is caused by excessive use of synthetic materials (curtains, carpets, floors and plastics), air-conditioning with synthetic filters, influence of positively charged TV or computer screens, use of laser printers and copy machines as well as polluted environment, smog and smoking. The influence of building constructions on the concentration of ions in interiors of buildings is significant, too.

Electronic microclimate of an interior is considerably changed in negative meaning by iron-concrete walls (panel houses) or by steel constructions. Such materials create a shading of an electric field of the Earth (Faraday cage) and thus strongly affect it. Consequently, they cause heightening of the unipolar coefficient which can harm the health. Positive ions can break into lungs at their high concentration. Inhaling can help ions to get into blood and body where they could cause unfavourable reactions, e.g. give off serotonin and histamine and thus cause tiredness, headache or make people feel drowsy, weary, and sleepy. Higher level of serotonin in blood reduces the lung capacity as well as ability of a body to absorb the oxygen. Serotonin could influence contraction of smooth muscles, which cause migraine, allergic reactions, neck ache, bronchus cough, abdominal cramps or a fit of heat. Pain of heart, allergies, hay fever, sickness and insomnia can be caused by rising level of histamine. Different equipments of air ionizers, based on electric principle, were developed in order to remove ionic non-balance of surrounding space which shows decreasing natural content of negative ions (anions). There are two types of ionizers - stable and portable. The stable ones use electric distribution network or any other generator of electric energy as a source of energy for the conditioning of oxygen. The portable ones are fed by battery or installed in cars where they are fed by the 12V voltage.

Stable ionizer solution according to EP 0 465 783 is well known.

Portable ionizer solutions according to PP 3469-92 CZ, or UV 5913 CZ are known, too. However, ionizers based on eclectic principle produce different amount of ozone and their functioning is unstable. After some time of their using the ratio of amount of generated negative ions and the ozone production fairly fluctuates. It is known that ozone breathing in higher concentrations is harmful to human body.

### The matter of invention

The inhaler of oxygen anions removes the problems of the present state.

The inhaler is filled with the medically conditioned oxygen enriched by negative onions. It consists of a container with a dosing valve, a doze controller, an inhaling cap and a shield cover. The container is closed with an upper neck in which a dosing valve is situated. It is created by a hollow closure that moves in a container. There is a pressure spring in the hollow of a case. Its one end leans against the circular ring of the bottom of the hollow of the case and the other one leans against the circular ring of the bottom of the closure. The closure has an embedding for sealing at the place of the neck of the container which is situated between an upper horizontal face of the upper neck of the container and a circular ring at the upper face of the case. There is at least one hole in the circular ring, situated vertically to the upper hollow of the closure. It is advantageous when there is a hole for attaching to dosing valve which is connected with the hollow of a circular ring finished with a face with nozzle. Inhaling cap consists of graded face with a hole for connection to dose controller and with at least one sucking air hole. It continues through enlarging conical part to a shape of a hollow cylinder with two opposite cut-outs for nose and chin. On the inner side of inhaling cap, at the place where conical part reaches hollow cylinder there are at least three outlets and three side stops fitted.

It is also advantageous if the cross section of dosing valve is rectangular. Its shorter side (inner) is modified by the outer rounding with the radius that equals to a half of the thickness of the packing.

The merit of the described solution is that the inhaler of oxygen anions enables to apply not only oxygen to human body but also lots of negative ions without any harmful amount of ozone and moreover without the source of energy either from electric net or from a battery.

Small-capacity inhaler of 0,8 l volume enables inhalation repeatedly in short intervals, 100 inhalations are possible from one content of a container.

### Survey Review of figures on drawings

The invention will be described and explained in the next paragraph, with the reference to drawings of inhaler of oxygen anions. The following figures show:

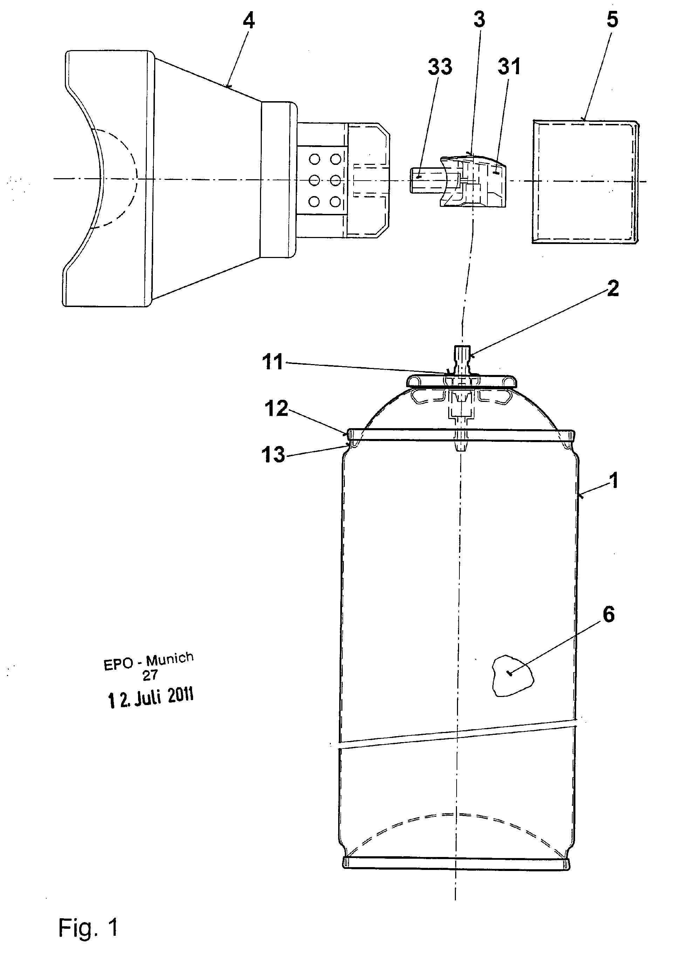

Figure 1: general view of details of the inhaler of oxygen anions

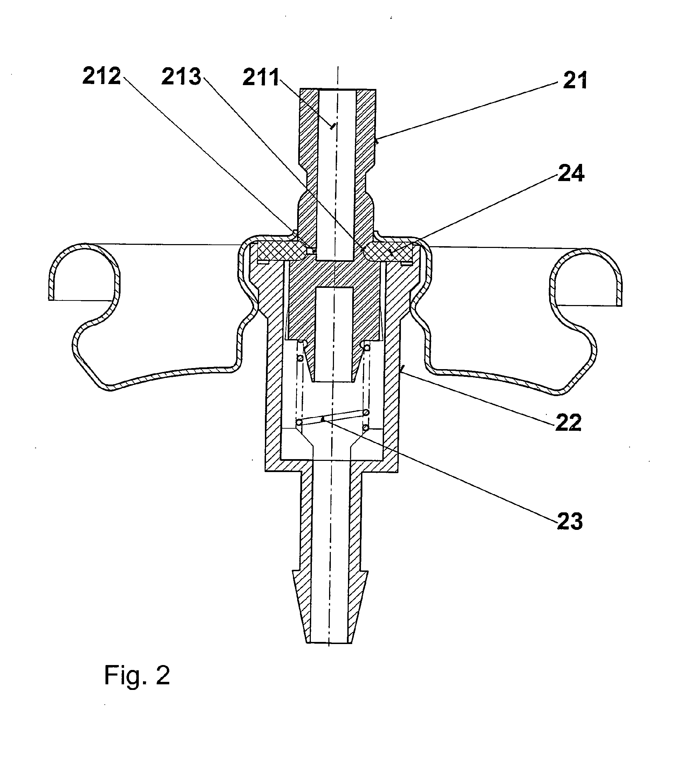

Figure 2: dosing valve in section

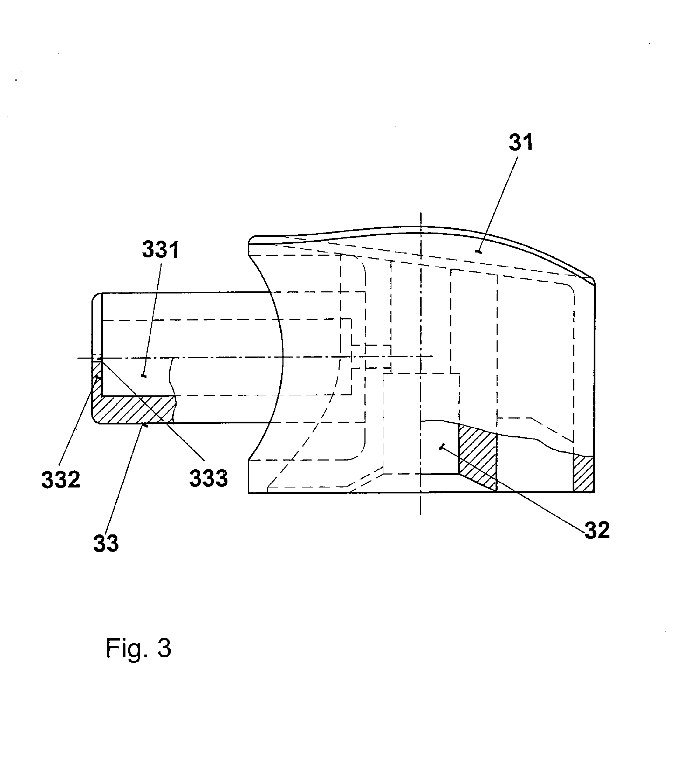

Figure 3: dosing controller in partial section

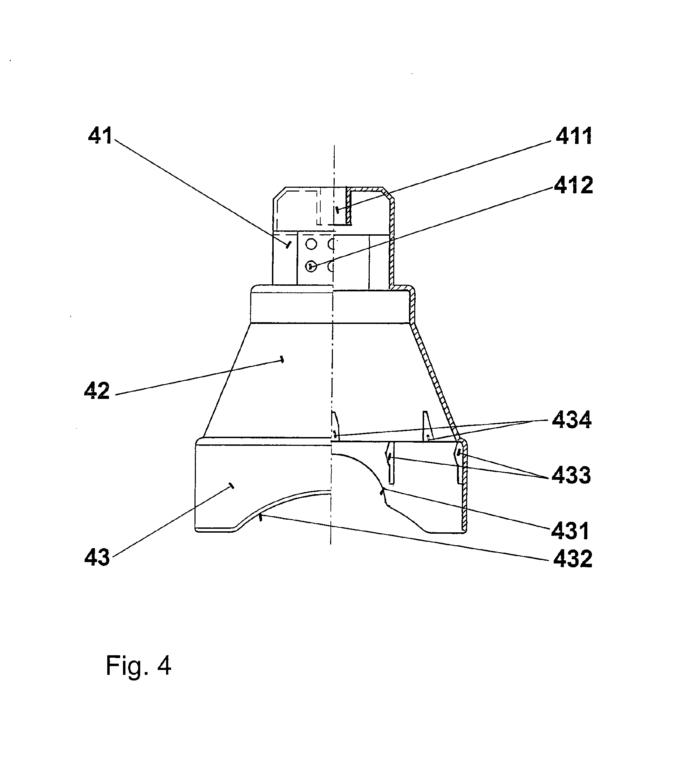

Figure 4: inhaling cap in partial section

### Examples of realization

The inhaler of oxygen anions filled with the medically conditioned oxygen enriched by negative onions in a physical-non-electrical way is shown in the pictures 1 to 4.

It consists of a container **1**, dosing valve **2**, dose controller **3**, inhaling cap **4** and shield cover **5**.

The container **1** is closed with an upper neck **11,** where dosing valve **2** is situated. In upper part under neck **11** there is upper repressing **12** with embedding **13.**

The dosing valve **2** consists of closure **21,** case **22,** pressure spring **23** and sealing **24.** The surface of the closure **21** is graded. There is a hollow **211** in its upper part which is connected to the surface by the hole **212** and it ends in the half-rounded embedding **213.** In the lower part of the closure **21** there is the relief hollow and the flat face. The pressure spring **23** contacts them with one ending and the other ending contacts the inner bottom of the case **22.** There is the recess at the upper face of the hollow case **22** where sealing **24** is placed. It presses hard upon the neck **11** of the container **1** and upon the embedding **213** of the closure **21.** The cross section of the sealing **24** is rectangular. Its shorter side (inner) is replaced by the outer rounding with the radius that equals to a half of the thickness of the sealing and by which the sealing **24** is pressed upon the embedding **213** of the dosing valve **2** closure **21.**

The dosing valve in the filled up container (in case of functional use of the inhaler of oxygen anions) works as a unidirectional valve. It is designed to seal perfectly when it is not used and when used for inhaling it should be operated by a minimum pressure on the dosing controller. At the same time it must fulfil the requirement of minimum repeated opening and closing of the container within 100 inhalations from the container of 0.8 l volume. The pressure spring secures a close state. The valve opens when there is a hand-operated pressure of the spring on the dose controller.

The dose controller **3** has a hole **32** in its body **31** to connect it with the dosing valve **2.** The hole **32** is connected with the hollow **331** of the cylindrical shoulder **33** by which the dose controller **3** is fixed to the inhaling cap **4.** The cylindrical shoulder **33** ends by a face **332** with a nozzle **333.**

The graded face **41** of inhaling cap **4** has a hole pointed towards its inside part. The graded face **41** continues through enlarging conical part to a shape of a hollow cylinder **43** with two opposite cut-outs **431** for nose **42** and chin. There are 6 holes **412** on surface of graded face **41** of inhaling cap **4.** The holes serve for mixing metered-dose oxygen anions with air while inhaling. Thus the absolute safety of the concentration of oxygen upon human body is secured. On the inner side of inhaling cap **4,** at the place where conical part **42** reaches hollow cylinder **43** there are 6 outlets **433** and 6 side stops **434** fitted. The shape of the cover **5** is that of a hollow cylinder which is closed by a face at one side.

Constructional arrangement of the inhaler of oxygen anions as it has been described keeps the container closed when filled with medium.

When applied, the dose controller **3** is put on by means of a hole **32** at upper part of closure **21** of dosing valve2. The inhaling cap **4** is put on cylindrical shoulder **33** of dose controller **3** by means of its hole **411.** Pressing down on dose controller 3 by a finger (on shaped part of its body **31)** the pressure is transferred on through closure **21** against the pressure of pressure spring **23.** Thus the hole **212** is released. Till then it was sealed by sealing **24.** Thus inner container **1** is connected to inhaling cap **4** and medium 6 (the medically conditioned oxygen enriched by negative onions in a physical-non-electrical way) starts to penetrate. Inhaling cap **4** is applied on nose and mouth and after simple pressure of dose controller **3** while breathing in, leakage of oxygen ions (anions) starts straight into breathing tract.

In store or transport position inhaling cap **4** is a part of hollow cylinder **43** that and is put on top of the container **1.** Its side stops **434** touch upper repressing **12** of container **1** and it is caught by outlets **433** at embedding **13** of container **1.** Cover **5** is fixed on graded face **41** of inhaling cap **4.**

### Industrial utilization

The inhaler of oxygen anions filled with the medically conditioned oxygen enriched by negative onions in a physical-non-electrical way enables compensation of a lack of physical and mental energy of a person, suppresses tiredness of human body at different activities, e.g. driving, moving in loading environment, staying in polluted environment, at stress situations, headaches, breathing problems, symptoms of various health problems. Large lot production of inhaler of oxygen anions is assumption of its wide industrial utilization.

## Claims

1. The inhaler of oxygen anions filled with the medically conditioned oxygen enriched by negative onions in a physical-non-electrical way, **characterized in that** consists of container (1) with a dosing valve (2), dosing controller (3), inhaling cap (4) and a cover (5) where the container (1) is closed by upper neck (11) in which the dosing valve (2) is situated. It is formed by hollow closure (21) tat moves in a case (22). In case hollow there is a pressure spring (23) that contacts circular ring of with the bottom of case hollow (22) by one ending and the by other ending it contacts the circular ring of the bottom of the closure (21). At the place of neck (11) of container (1) there is closure (21) embedding (213) for sealing (24) which is situated between upper horizontal area of upper neck (11) of container (1) and embedding at upper face of case (22). There is at least one hole (212) entering upper hollow (211) of closure (2) in embedding (213) vertical to upper hollow (211) of closure (21).

2. The inhaler of oxygen anions according to claim 1, **characterized in that** the dose controller (3) has a hole (32) of connection to a dosing valve which is connected with hollow (331) of the cylindrical shoulder (33) that ends by a face (332) and a nozzle (333).

3. The inhaler of oxygen anions according to claim 1 or 2, **characterized in that** the inhaling cap (4) consists of graded face (41) with a hole (411) to connect to outing of a dose controller (3) and to at least one hole (412) for air sucking, then there is an enlarging conical part (42) that reaches hollow cylinder (43) with two opposite cut-outs (431) for nose and chin. At the inner side of the place where conical part reaches hollow cylinder there are at least three outlets (433) and three side stops (434).

4. The inhaler of oxygen anions according to claim 1 or claim 2 or claim 3, **characterized in that** the cross section of the circular sealing (24) of dosing valve (2) is rectangular. Its shorter, inner side is modified by outer rounding with radius that equals to a half of thickness of sealing.
